# EUROPÄISCHE PATENTSCHRIFT

(11) **EP 1 063 217 B1**
(45) Veröffentlichungstag und Bekanntmachung des Hinweises auf die Patenterteilung: **05.03.2003**
(21) Anmeldenummer: 00111532.8
(22) Anmeldetag: 30.05.2000
(51) Int. Cl.: C07C 45/34, C07C 45/86, C07C 49/603

(54) **Verfahren zur Herstellung von Oxoisophoron in Gegenwart eines oder mehrerer Azetatsalze**
Process for preparing oxoisophorone in presence of one or more acetate salts
Procédé de préparation d'oxoisophorone en présence d'un ou plusieurs sels d'acétate

(30) Priorität: 25.06.1999 DE 19929362
(43) Veröffentlichungstag der Anmeldung: 27.12.2000
(73) Patentinhaber: BASF AKTIENGESELLSCHAFT, 67056 Ludwigshafen (DE)
(72) Erfinder: Klatt, Martin Jochen, Dr., 67098 Bad Dürkheim (DE); Müller, Thomas, Dr., 67246 Dirmstein (DE); Bockstiegel, Bernhard, Dr., 67354 Römerberg (DE)

(56) Entgegenhaltungen:
- EP-A- 0 808 816
- DE-A- 2 610 254

## Beschreibung

Die vorliegende Erfindung betrifft ein Verfahren zur Herstellung von 3,5,5-Trimethylcyclohex-2-en-1,4-dion (Oxoisophoron; OIP) durch Oxidation von 3,5,5-Trimethyl-cyclohex-3-en-1-on (β-Isophoron, β-IP) mit molekularem Sauerstoff in Gegenwart eines Lösungsmittels, einer Base und einem Übergangsmetall-Salenderivat als Katalysator.

Oxoisophoron (OIP) kann als Geschmacks- oder Riechstoff in Lebensmitteln oder in kosmetischen Formulierungen verwendet werden. OIP ist darüberhinaus ein Zwischenprodukt für die Herstellung von Vitaminen und Carotinoiden.

Es ist bekannt, OIP durch Oxidation von β-Isophoron (β-IP) mit molekularem Sauerstoff in Gegenwart eines inerten Lösungsmittels, einer Base und eines Mn- oder Co-Salenderivats herzustellen. Die Patentschrift DE 2610254 C2 beschreibt die Verwendung einer Vielzahl von Cobalt(II)- und Mangan(II)salen-Derivaten als Katalysatoren, wobei die salenartigen Chelatliganden aus einer Reihe von Diaminen und Hydroxycarbonylverbindungen hergestellt werden können. In der Liste der Hydroxycarbonylverbindungen werden neben vielen anderen auch halogenierte 2-Hydroxybenzaldehyde mit einem weit variierbaren Substitutionsmuster erwähnt. Mit diesem Verfahren werden insbesondere bei Verwendung von unsubstituierten aromatischen Mn- uns Co-Salenen (Liganden hergestellt aus verschiedenen Diaminen und 2-Hydroxy-benzaldehyd) oftmals nur geringe Umsätze, Ausbeuten und Selektivitäten erreicht. Eine Substitution am Aromaten mit elektronenziehenden Resten, wie die Einführung einer Nitro-gruppe führt, wie in Beispiel 5 *loc. cit.* gezeigt, zu einer geringeren Ausbeute.

In JP 01090150 werden in einem analogen Verfahren aromatische Mangan(III)salen-Derivate mit einer weiten Variationsmöglichkeit im Substitutionsmuster, den Substituenten, dem Gegenion und der Anzahl der C-Atome in der Aminbrücke als Katalysatoren beschrieben. Als bestes Ergebnis werden unter der Verwendung eines chlorierten Mn(III)salens mit X = Acetat als Gegenion bei geringen Eduktkonzentrationen Ausbeuten bis zu 90,7% erreicht. Aufgrund der geringen Eduktkonzentrationen ist die Raum-Zeit-Ausbeute aber ebenfalls gering.

EP 0808816 A1 beschreibt ein verbessertes Verfahren mit Zusatz katalytischer Additive wie organischer Säuren, aliphatischer Alkohole, Verbindungen, die eine Enolstruktur ausbilden können und Lithiumsulfat. Dadurch sollen auch bei höheren Eduktkonzentrationen gute Selektivitäten und damit höhere Raum-Zeit-Ausbeuten erreicht werden. Ausgehend von Eduktkonzentrationen von 1,4 mol/l werden durch die Zusätze Selektivitäten bis zu 92 % (Acetylaceton) bei 80% Umsatz erreicht. Das Additiv Essigsäure bewirkt hier die größte Reaktionsgeschwindigkeit (5,3*10⁻² l/min). Das Verfahren hat den Nachteil, daß die erzielten Raum-Zeit-Ausbeuten nur im Labormaßstab erreicht werden können, da im großtechnischen Maßstab große Edukttonagen bei exothermen Reaktionen mit molekularem Sauerstoff nicht vorgelegt werden können, sondern langsam (bis zu 4 Stunden) dem Reaktionsgemisch zugeführt werden müssen, um eine Explosionsgefahr zu vermeiden. Ein nur 80%-iger Umsatz erfordert weiterhin eine aufwendige Abtrennung der noch nicht umgesetzten Edukte.

Bei allen Verfahren des Standes der Technik entstehen neben Hochsiedern, die in der Aufreinigung weniger stören, die folgenden, identifizierten Nebenprodukte IV (α-Isophoron), V, VI und VII, die eine Aufreinigung oder eine weitere chemische Umsetzung des OIP erschweren:

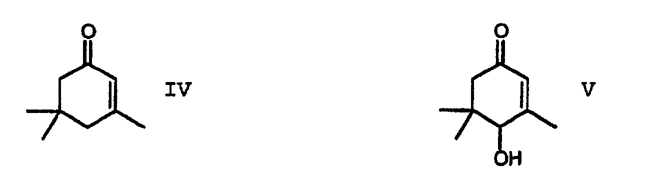

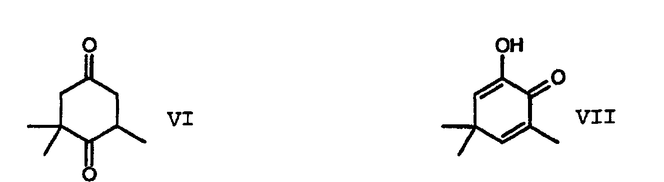

α-Isophoron (IV) entsteht bei den Verfahren nach dem Stand der Technik mit bis zu 3,2% Ausbeute und Verbindung V mit bis zu 4,4 % Ausbeute. Besonders störend für weitere Synthesen ist die Verbindung VI, da sie ein ähnliches chemisches Verhalten zeigt wie das Produkt OIP.

Neben den geringen Ausbeuten bei steigender Eduktkonzentration haben alle Verfahren des Standes der Technik den weiteren Nachteil gemeinsam, daß sie bereits unter simulierten großtechnischen Bedingungen bezüglich Temperatur und Sauerstoffverbrauch einem unregelmäßigen Reaktionsverlauf zeigen, so daß starke Schwankungen während der Reaktion überwacht und ausgeglichen werden müssen.

Alle bekannten Verfahren verwenden ferner aus der Vielzahl der Kombinationsmöglichkeiten an Basen und Lösungsmitteln Triethylamin als Base, mehrheitlich kombiniert mit Diglyme (Dimethyldiglykol) als Lösungsmittel. Da dieses Gemisch eine Zündtemperatur von 0°C aufweist, lassen sich die bekannten Verfahren auch aus diesem Grund nur unter hohem Sicherheitsaufwand im großtechnischen Maßstab durchführen.

Ferner zeigen die bekannten Verfahren ein langsames Anspringverhalten der Reaktion, so daß sich die Konzentration des kontinuierlich eingeleiteten Edukts β-IP zu Beginn hochpegelt. Daraus ergibt sich, insbesondere im großtechnischen Maßstab, der Nachteil, daß aus Sicherheitsgründen zunächst eine geringere Eduktmenge zugeleitet werden kann, was in einer geringeren Raum-Zeit-Ausbeute resultiert. Zusätzlich führt ein erhöhter β-Isophoronanteil zu einer erhöhten Rückisomerisierung zu α-Isophoron, was in eine geringere Selektivität an β-Isophoron resultiert.

Es stellte sich daher die Aufgabe, den geschilderten Mängeln abzuhelfen und ein Verfahren zu entwickeln, das selbst bei hohen Eduktkonzentrationen gute Ausbeuten, Selektivitäten und Raum-Zeit-Ausbeuten liefert, die sich auch in den großtechnischen Maßstab übertragen lassen. Des weiteren sollte die Entstehung der Nebenprodukte IV, V, VI und VII und die Aufpegelung des β-IP zu Beginn der Reaktion reduziert werden.

Demgemäß wurde ein Verfahren gefunden, indem man 3,5,5-Trimethylcyclohex-2-en-1,4-dion durch Oxidation von 3,5,5-Trimethyl-cyclohex-3-en-1-on mit molekularem Sauerstoff in Gegenwart eines Lösungsmittels, einer Base und einem Katalysator der allgemeinen Formel I herstellt, 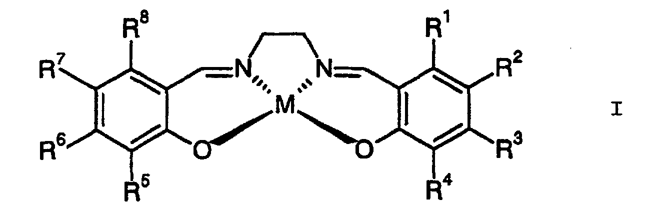 wobei
- R¹, R², R³, R⁴, R⁵, R⁶, R⁷ und R⁸: unabhängig voneinander Wasserstoff, Halogen, NO₂, COR⁹, OCOR⁹, COOR⁹_{,} SO₂R⁹ oder SO₃R⁹ bedeuten, wobei
- R⁹: Wasserstoff oder einen C₁-C₄-Alkylrest bedeutet,
- M: Mn(II), Mn(III)⁽⁺⁾X⁽⁻⁾, Co(II), Co(III)⁽⁺⁾X⁽⁻⁾, Fe(II), Fe(III)⁽⁺⁾X⁽⁻⁾, Cu(II) oder Ru(II) bedeutet, wobei
- X⁽⁻⁾: bei Metallen M in der Oxidationsstufe III ein negativ geladenes Gegenion darstellt,
dadurch gekennzeichnet, daß man das Verfahren in Gegenwart eines oder mehrerer Acetatsalze der allgemeinen Formel II

(R¹⁰R¹¹R¹²C-COO⁽⁻⁾)ₘ Y^{(m+)} (II)

als Additiv durchführt, wobei
- R¹⁰, R¹¹ und R¹²: unabhängig voneinander Wasserstoff, F, Cl, Br, I oder einen C₁-C₄-Alkylrest,
- Y: NH₄⁺ oder ein ein- bis vierfach geladenes Metallkation und
- m: 1, 2, 3 oder 4 bedeuten.

Ausgangsverbindung des Verfahrens ist 3,5,5-Trimethyl-cyclohex-3-en-1-on (β-Isophoron; β-IP). Die Umsetzung zu 3,5,5-Trimethylcyclohex-2-en-1,4-dion (Oxoisophoron; OIP) erfolgt in einem Lösungsmittel durch Oxidation mit molekularem Sauerstoff in Gegenwart einer Base, dem vorstehend erwähnten Katalysator der Formel I und Acetatsalzen der allgemeinen Formel II als Additive.

Die Katalysatoren der Formel I sind Metall-Salenkomplexe oder davon abgeleitete Metall-Salenderivate, die aus einem zentralen Metallatom und einem vierzähnigen Chelatliganden (Salen-Liganden) aufgebaut sind. Der Salenligand kann in an sich bekannter Weise aus Ethylendiamin und den entsprechenden, gegebenenfalls mit R¹ bis R⁸ substituierten Salicylaldehyden hergestellt werden.

Unter M werden die Metalle Mn, Co, Fe, Cu oder Ru in der Oxidationsstufe II oder III verstanden. Aus Gründen des Ladungsausgleichs tragen die Metalle in der Oxidationsstufe III im Komplex ein negativ geladenes Gegenion X⁽⁻⁾. Als negativ geladene Gegenionen X⁽⁻⁾ seien beispielhaft Halogenide wie Cl⁽⁻⁾, Br⁽⁻⁾, I⁽⁻⁾, C₁-C₅-Alkanoate wie Acetat, Propiat oder weitere Anionen wie N₃⁽⁻⁾, Thiocyanat, Cyanat, Isothiocyanat, BF₄⁽⁻⁾, PF₆⁽⁻⁾, SO₄²⁽⁻⁾, PO₄³⁽⁻⁾ oder NO₃⁽⁻⁾ erwähnt. Bevorzugte Metalle bzw. Metalle mit Gegenion sind Co(II), Mn(II), oder Mn(III)Cl⁽⁻⁾.

Der aromatische Rest des Salenliganden kann keine Substituenten (R¹ = R² = R³ = R⁴ = R⁵ = R⁶ = R⁷ = R⁸ = Wasserstoff) tragen oder mit den Resten R¹ bis R⁸ unabhängig voneinander mit Halogen, wie F, Cl, Br oder I oder NO₂, COR⁹, OCOR⁹, COOR⁹, SO₂R⁹ oder SO₃R⁹ substituiert sein. R⁹ kann Wasserstoff oder einen C₁-C₄-Alkylrest wie Methyl, Ethyl, n-Propyl, i-Propyl, n-Butyl, i-Butyl oder t-Butyl bedeuten.

In bevorzugten Salenliganden stellen R¹, R³, R⁶ und R⁸ Wasserstoff dar, während die Reste R² und R⁷ die vorstehend erwähnten Substituenten, und R⁴ und R⁵ Wasserstoff oder die vorstehend erwähnten Substituenten bedeuten. Besonders bevorzugt sind Salenliganden mit R¹ = R³ = R⁶ = R⁸ = Wasserstoff, R² = R⁷ = Cl und R⁴ = R⁵ = Wasserstoff oder Cl.

Bevorzugte Katalysatoren sind dementsprechend Metall-Salene mit Co(II), Mn(II) oder Mn(III)Cl⁽⁻⁾ als zentrales Metall bzw. Metall mit Gegenion und Salenliganden mit R¹ = R³ = R⁶ = R⁸ = Wasserstoff, R² = R⁷ = Halogen, NO₂, COR⁹, OCOR⁹, COOR⁹_{,} SO₂R⁹ oder SO₃R⁹ und R⁴ = R⁵ = Wasserstoff oder Halogen, NO₂, COR⁹, OCOR⁹, COOR⁹, SO₂R⁹ oder SO₃R⁹. Besonders bevorzugt sind Metall-Salene mit Mn(II) oder Mn(III)Cl⁽⁻⁾ als zentrales Metall bzw. Metall mit Gegenion und Salenliganden mit R¹ = R³ = R⁶ = R⁸ = Wasserstoff, R² = R⁷ = Cl und R⁴ = R⁵ = Wasserstoff oder Cl.

Das Verfahren wird in Gegenwart eines oder mehrerer Acetatsalze der allgemeinen Formel II als Additiv durchgeführt. Mehrere Acetatsalze bedeutet eine Mischung, enthaltend zwei oder drei Acetatsalze. Die Reste R¹⁰, R¹¹ und R¹² können dabei unabhängig voneinander Wasserstoff, F, Cl, Br, I oder einen C₁-C₄-Alkylrest, wie vorstehend für R⁹ erwähnt, bedeuten.

Y stellt ein positiv geladenes Gegenion, wie NH₄⁺ oder ein Metallkation der 1.-4. Hauptgruppe, wie Li⁺, Na⁺, K⁺, Ca²⁺, oder Mg²⁺ dar. Der Parameter m stellt im Acetatsalz den Acetatkoeffizienten entsprechend der Ladungszahl des positiv geladenen Gegenions dar und kann 1, 2, 3 oder 4 betragen. Bevorzugte Acetatsalze sind Y-Acetate oder Y-Halogenacetate.

Besonders bevorzugte Acetatsalze sind LiOAc (Lithiumacetat), NaOAc (Natriumacetat), KOAc (Kaliumacetat), NH₄OAc (Ammoniumacetat), Ca(OAc)₂ (Kalziumacetat), Mg(OAc)₂ (Magnesiumacetat), Lithiumtrifluoracetat oder Lithiumdichloracetat.

Unter Lösungsmittel werden organische Lösungsmittel und Wasser verstanden. Beispielhaft seien für organische Lösungsmittel gegebenenfalls halogenierte aliphatische oder aromatische Kohlenwasserstoffe, wie Pentan, Hexan, technisches Hexan, Heptan, Benzol, Toluol, Xylol, technisches Xylol, Methylenchlorid, Chloroform, Ether, wie Dimethylether, Diethylether, THF, Dioxan, Diglykol, Dimethyldiglykol(Diglyme), Alkohole, wie Methanol, Ethanol, Propanol, Ketone, wie Aceton, Ester, wie Essigsäureethylester, Nitrile, wie Acetonitril oder Carboxamide, wie Dimethylformamid (DMF), Dimethylacetamid (DMA), N-Methylpyrrolidon (NMP) verstanden.

Bevorzugt sind organische Lösungsmittel mit Metall-komplexierenden Eigenschaften, also organische Lösungsmittel, die Gruppen oder Atome mit Elektronen-Donor-Eigenschaften enthalten, wie NMP, DMF oder DMA.

Aufgrund der höheren Zündtemperatur ihrer Mischungen mit nachstehenden Basen, insbesondere Tripropylamin, sind DMF, DMA oder NMP als organische Lösungsmittel besonders bevorzugt.

Unter Basen werden Brönsted-Basen wie LiOH, NaOH, KOH, Li-, Naoder K-Alkoholate oder quartäres Ammoniumhydroxid oder

C₂-C₁₄-Dialkylamine, wie Dimethylamin, Diethylamin, Methylethylamin, Dipropylamin, Diisopropylamin, Ethylbutylamin, Dibutylamin oder C₃-C₂₀-Trialkylamine, wie Trimethylamin, Triethylamin, Tripropylamin oder Tributylamin verstanden.

Bevorzugte Basen sind C₃-C₂₀-Trialkylamine.
Aufgrund der höheren Zündtemperatur der Mischung mit DMF oder DMA ist die Verwendung von Tripropylamin als Base besonders bevorzugt.

Die Kombinationen Tripropylamin als Base und DMF oder DMA als Lösungsmittel stellen deshalb eine bevorzugte Ausführungsform dar.

Im erfindungsgemäßen Verfahren beträgt die Konzentration des Edukts β-IP typischerweise 0,5 bis 4,0 mol/l, insbesondere 2,5 bis 3,6 mol/l.

Die Temperatur der Reaktion wird typischerweise auf 20°C geregelt, um die stark exotherme Reaktion kontrolliert ablaufen zu lassen.

Die Konzentrationen der Reagenzien und Katalysatoren sind nicht kritisch und betragen typischerweise jeweils für den Katalysator und die Acetatsalze oder die Mischung der Acetatsalze als Additive 0,2 mol-% bis 5 mol-%, insbesondere 0,3 mol-% bis 0,7 mol-%, für die Base 0,1 mol/l bis 0,5 mol/l, insbesondere 0,15 mol/l bis 0,35 mol/l.

Die Dauer der Umsetzung beträgt typischerweise 0,1 bis 10 Stunden, insbesondere 4 bis 8 Stunden.

Besonders hohe Ausbeuten, Selektivitäten, Raumzeitausbeuten und schnelles Anspringen der Reaktion und damit niedrige Aufpegelungen an β-IP, auch bei hohen Eduktkonzentrationen, werden durch nachstehende, bevorzugte Ausführungsformen erreicht.

Dabei führt man das Verfahren in Gegenwart eines Katalysators der allgemeinen Formel Ia durch, 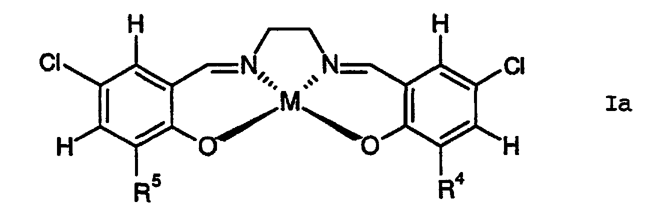 wobei
- R⁴ und R⁵: unabhängig voneinander Wasserstoff oder Cl und
- M: Mn(II) oder Mn(III)⁽⁺⁾ Cl⁽⁻⁾ bedeuten,
unter Verwendung von Dimethylformamid (DMF) oder Dimethylacetamid (DMA) als Lösungsmittel, Tripropylamin als Base und unter Zusatz von NH₄OAc, LiOAc, NaOAc oder einer Mischung dieser Acetate als Additiv.

Um eine weitere Erhöhung der Konzentration an β-IP und damit eine weitere Erhöhung der Raum-Zeit-Ausbeute zu ermöglichen, wird in einer besonders bevorzugten Ausführungsform das Verfahren unter inverser Reaktionsführung, d. h. unter langsamer, kontinuierlicher Zuführung von β-IP in das Reaktionsgemisch durchgeführt. Typischerweise beträgt die Dauer der Zuführung zwischen einer und sechs Stunden, insbesondere 4 Stunden.

Das Verfahren zeichnet sich gegenüber dem Stand der Technik durch folgende Vorteile aus:
- Das Verfahren ist auch im großtechnischen Maßstab wirtschaftlich, d. h. mit hohen Raum-Zeit-Ausbeuten durchführbar.
- Es werden auch bei hohen Eduktkonzentrationen an β-IP (3,5 mol/l) bei einem Umsatz von 100% Selektivitäten und damit auch Ausbeuten bis zu 90% erreicht. Dadurch wird die Raum-Zeit-Ausbeute erhöht. Die Selektivitätserhöhung gelingt durch eine Reduzierung der normalerweise schwierig abzutrennenden Nebenprodukte IV-VII.
- Die Reaktion springt schnell an, was bewirkt, daß die Aufpegelung an β-IP zu Beginn der Reaktion reduziert wird. Dies hat einerseits den sicherheitstechnischen Vorteil einer geringeren Explosionsgefahr, andererseits den wirtschaftlichen Vorteil einer höheren Selektivität und Ausbeute, da das Nebenprodukt α-Isophoron durch Rückisomerisierung von β-IP entsteht. Des weiteren kann eine höhere Eduktmenge zugeleitet bzw. die gleiche Eduktmenge schneller zugeleitet werden. Die Erhöhung der Eduktmenge oder der Geschwindigkeit führt zu einer Erhöhung der Raum-Zeit-Ausbeute. Durch den Zusatz der Additive kann zusätzlich die Gesamtreaktionszeit von 8 Stunden auf 6 Stunden reduziert werden.
- Die Reaktion verläuft in Bezug auf Temperatur und Sauerstoffverbrauch sehr regelmäßig, so daß die Parameter nur zu Beginn der Reaktion eingestellt und nicht überwacht und nachgeregelt werden müssen. Dies ist als weiterer sicherheitstechnischer Vorteil zu sehen.
- Durch geeignete Wahl der Base und des Lösungsmittels kann die Zündtemperatur des Reaktionsgemisches erhöht werden, was einen weiteren sicherheitstechnischen Vorteil bietet.

Die nachstehenden Beispiele erläutern die Erfindung.

### Beispiel 1 bis 3

### Herstellung der Katalysatoren

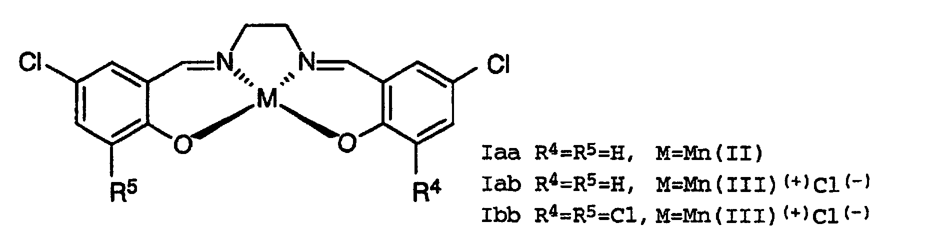

### Beispiel 1

### Herstellung des Katalysators der Formel Iaa

6,75 g (20 mmol) der Schiff'schen Base (Salenligand, hergestellt aus Ethylendiamin und 5-Chlor-2-Hydroxy-Benzaldehyd) wurden unter Stickstoff in Natronlauge (1,6 g NaOH auf 100 ml Wasser) bei 80 bis 85°C gelöst. Zu dieser Lösung tropfte man innerhalb von 30 Minuten eine Lösung aus 3,38 g (20 mmol) MnSO₄.H₂O in 15 ml Wasser und rührte bis zum vollständigen Umsatz der Schiff'schen Base (ca. 2 bis 3 h; DC-Kontrolle; Cyclohexan/Essigester 2:1) bei 85°C nach.

Die Reaktionsmischung wurde auf 10°C gekühlt, der orange-braune Niederschlag abfiltriert und mit Wasser neutralisiert. Das Produkt wurde bis zur Gewichtskonstanz bei 50°C im Trockenschrank getrocknet.

Ausbeute: 7,4 - 7,7 g (94 bis 99 % der Theorie)

### Beispiel 2

### Herstellung des Katalysators der Formel Iab

6,75 g (20 mmol) der entsprechenden Schiff'schen Base (Salenligand, hergestellt aus Ethylendiamin und 5-Chlor-2-Hydroxy-Benzaldehyd) wurden in 150 ml siedendem Ethanol gelöst, mit 5,36 g Mn(OAc)₃.2 H₂O (20 mmol) versetzt und drei Stunden unter Rückfluß gehalten. Anschließend wurden drei Äquivalente Lithiumchlorid (2,54 g, 60 mmol) zugegeben und die Reaktionslösung weitere drei Stunden gekocht. Nach Abkühlen auf Raumtemperatur wurde der braunschwarze Feststoff abfiltriert und mit MTB-Ether (100 ml) gewaschen. Bei 50°C wurde der Komplex über Nacht im Vakuum getrocknet.

Ausbeute: 8,40 g (99 % der Theorie)

### Beispiel 3

### Herstellung des Katalysators der Formel Ibb

Herstellung analog zu Beispiel 3, mit dem Unterschied, daß ein Salenligand, hergestellt aus Ethylendiamin und 3,5-Dichlor-2-Hydroxy-Benzaldehyd verwendet wurde.

Ausbeute: 95 bis 99 % der Theorie

### Beispiele 4 bis 7

### Herstellung von OIP durch Oxidation von β-IP mit Zusatz eines Additivs

520 ml DMF bzw. DMA (Dimethylacetamid), 28,6 g Tripropylamin (0,2 mol) und etwa ein Drittel der benötigten Menge an Katalysator und Additiv (Li- bzw. Ammoniumacetat) (total: je 9 mmol, 0,45 mol-%) wurden in einen 1-L-HWS-Glasreaktor vorgelegt und auf 20°C temperiert. Unter Sauerstoffbegasung (mit Abgasführung) wurden innerhalb von 4 Stunden unter Temperaturkontrolle (20 +/- 1°C) 276 g β-Isophoron (2,0 mol) zugetropft. Die restliche Menge des Katalysators und Additivs (Li- bzw. Ammoniumacetat) wurde in DMF bzw. DMA suspendiert und nach zwei, vier bzw. sechs Stunden portionsweise zudosiert. Die Gesamtbegasung betrug acht Stunden.

Die Ausbeuten an Oxoisophoron wurden gaschromatographisch unter Verwendung eines internen Standards (Methylbenzoat) bestimmt. Neben der Ausgangsverbindung β-isophoron und dem Produkt Oxoisophoron wurde die Menge der entstandenen Nebenprodukte IV, V, VI und VII gaschromatographisch durch Flächenintegration bestimmt

Die Reaktion wurde mit verschiedenen Katalysatoren und Lösungsmitteln durchgeführt. Die jeweiligen Reaktionsbedingungen und Ergebnisse sind in Tabelle 1 zusammengestellt.

### Vergleichsbeispiele 1 und 2

### Herstellung von OIP ohne Zusatz von Additiven.

Die Herstellung von OIP mit den Katalysatoren der Formel Ica und Icb erfolgte ohne Zusatz von Additiven.

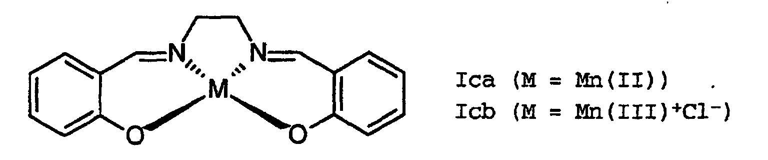

Dazu wurden 520 ml DMF 28.6 g Tripropylamin (0.2 mol) und etwa ein Drittel der benötigten Menge an Katalysator (total: je 9 mmol, 0.45 mol-%) in einen 1-L-HWS-Glasreaktor vorgelegt und auf 20°C temperiert. Unter Sauerstoffbegasung (mit Abgasführung) wurden innerhalb von 4 Stunden unter strikter Temperaturkontrolle (20 +/- 1°C) 276 g β-Isophoron (2.0 mol) zugetropft. Die restliche Menge des Katalysators wurde in DMF bzw. Dimethylacetamid suspendiert und nach zwei, vier bzw. sechs Stunden portionsweise zudosiert. Die Gesamtbegasung betrug acht Stunden. Die Analytik erfolgte analog zu den Beispielen 5-12. Die Reaktionsbedingungen und Ergebnisse sind in Tabelle 2 zusammengefaßt.

### Beispiele 8 und 9 und Vergleichsbeispiel 3

### Kinetischer Verlauf der β-IP-Konzentration ohne und mit Zugabe von Additiven

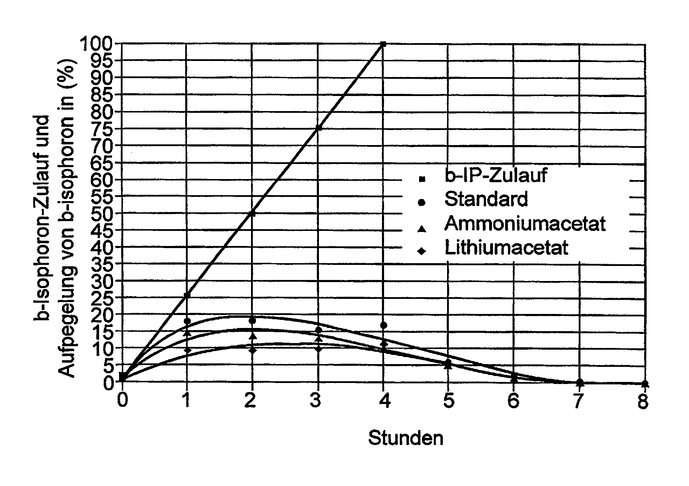

Die Umsetzung wurde analog zu den Beispielen 4 bis 7 und den Vergleichsbeispielen 1 und 2 einmal ohne Additive und zweimal jeweils mit dem Additiv Ammoniumacetat und Lithiumacetat unter Verwendung des Katalysators Ica durchgeführt. Während der Umsetzung wurde die Aufpegelung an β-Isophoron im Reaktionsgefäß kinetisch verfolgt. Die Ergebnisse sind in Tabelle 3 gezeigt und in Abbildung 1 grafisch aufgetragen. Die Additive hemmen die Aufpegelung von β-Isophoron.

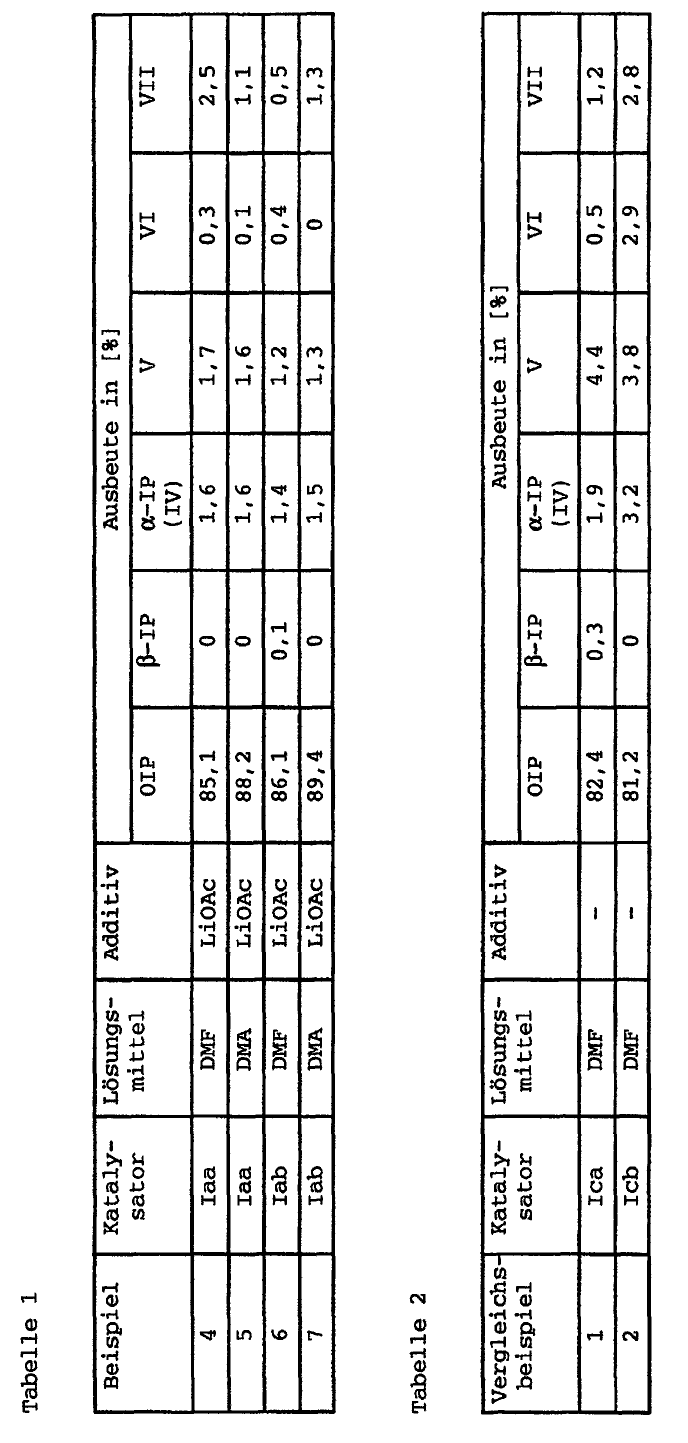

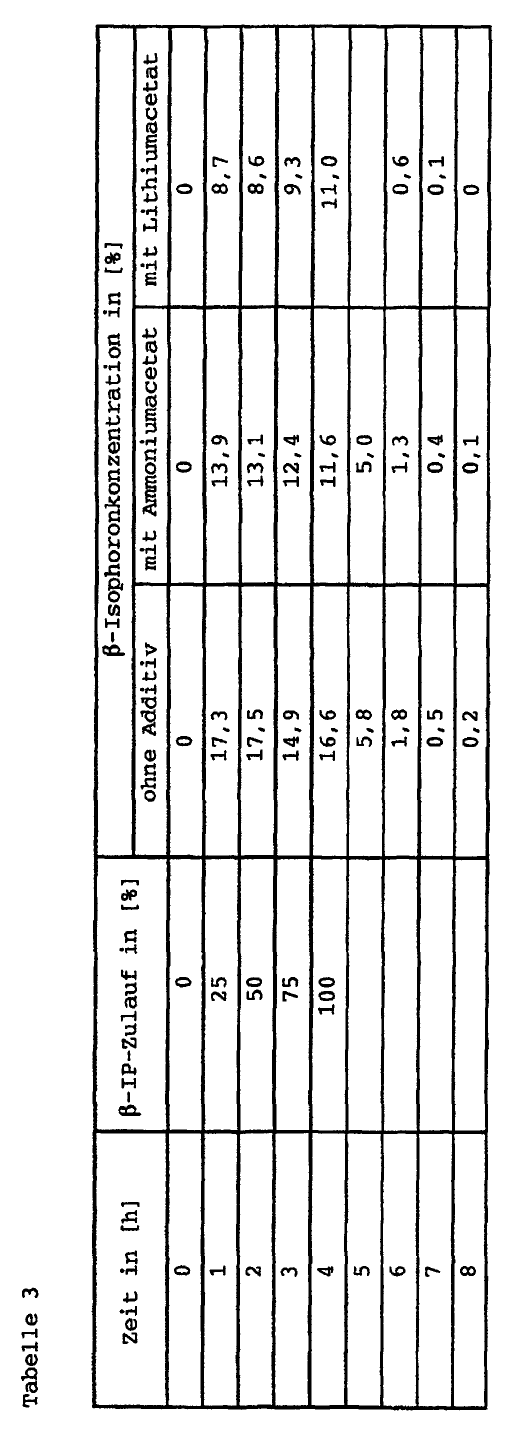

## Patentansprüche

1. Verfahren zur Herstellung von 3,5,5-Trimethylcyclohex-2-en-1,4-dion durch Oxidation von 3,5,5-Trimethyl-cyclohex-3-en-1-on mit molekularem Sauerstoff in Gegenwart eines Lösungsmittels, einer Base und einem Katalysator der allgemeinen Formel I, 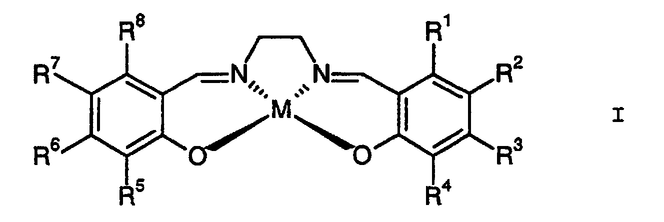 wobei
R¹, R², R³, R⁴, R⁵, R⁶, R⁷ und R⁸ unabhängig voneinander Wasserstoff, Halogen, NO₂, COR⁹, OCOR⁹, COOR⁹_{,} SO₂R⁹ oder SO₃R⁹ bedeuten, wobei
R⁹ Wasserstoff oder einen C₁-C₄-Alkylrest bedeutet,
M Mn(II), Mn(III)⁽⁺⁾X⁽⁻⁾, Co(II), Co(III)⁽⁺⁾X⁽⁻⁾, Fe(II), Fe(III)⁽⁺⁾X⁽⁻⁾, Cu(II) oder Ru(II) bedeutet, wobei
X⁽⁻⁾ bei Metallen in der Oxidationsstufe III ein negativ geladenes Gegenion darstellt,
**dadurch gekennzeichnet, daß** man das Verfahren in Gegenwart eines oder mehrerer Acetatsalze der allgemeinen Formel II
(R¹⁰R¹¹R¹²C-COO⁽⁻⁾)ₘ Y^{(m+)} (II)
als Additiv durchführt, wobei
R¹⁰, R¹¹ und R¹² unabhängig voneinander Wasserstoff, F, Cl, Br, I oder einen C₁-C₄-Alkylrest,
Y NH₄⁺ oder ein ein- bis vierfach geladenes Metallkation der 1. bis 4. Hauptgruppe und
m 1, 2, 3 oder 4 bedeuten.

2. Verfahren nach Anspruch 1, **dadurch gekennzeichnet, daß** man als Additiv NH₄OAc, LiOAc, NaOAc, KOAc oder eine Mischung dieser Acetate verwendet.

3. Verfahren nach einem der Ansprüche 1 oder 2, **dadurch gekennzeichnet, daß** man als Lösungsmittel ein Lösungsmittel mit komplexierenden Eigenschaften verwendet.

4. Verfahren nach einem der Ansprüche 1 oder 2, **dadurch gekennzeichnet, daß** man als Lösungsmittel Dimethylformamid (DMF), N-Methylpyrrolidon (NMP) oder Dimethylacetamid (DMA) verwendet.

5. Verfahren nach einem der Ansprüche 1 bis 4, **dadurch gekennzeichnet, daß** man als Base ein Trialkylamin mit 3 bis 20 C-Atomen verwendet.

6. Verfahren nach einem der Ansprüche 1 bis 4, **dadurch gekennzeichnet, daß** man als Base Tripropylamin verwendet.

7. Verfahren nach Anspruch 1 oder 2, **dadurch gekennzeichnet, daß** man als Lösungsmittel Dimethylformamid (DMF) oder Dimethylacetamid (DMA) in Kombination mit Tripropylamin als Base verwendet.

8. Verfahren nach Anspruch 1, **dadurch gekennzeichnet, daß** man das Verfahren in Gegenwart eines Katalysators der allgemeinen Formel Ia 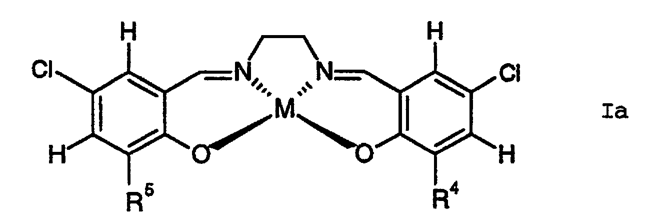 durchführt, wobei
R⁴ und R⁵ unabhängig voneinander Wasserstoff oder Cl
M Mn(II) oder Mn(III)⁽⁺⁾ Cl⁽⁻⁾ bedeuten,
unter Verwendung von Dimethylformamid (DMF) oder Dimethylacetamid (DMA) als Lösungsmittel,
Tripropylamin als Base und
unter Zusatz von NH₄OAc, LiOAc, NaOAc oder einer Mischung dieser Acetate als Additiv.

9. Verfahren nach Anspruch 1, **dadurch gekennzeichnet, daß** man das Verfahren unter inverser Reaktionsführung durch Zuleiten des Edukts zur Reaktionsmischung, enthaltend das Lösungsmittel, die Base und den Katalysator, durchführt.

## Claims

1. A process for preparing 3,5,5-trimethylcyclohex-2-ene-1,4-dione by oxidation of 3,5,5-trimethylcyclohex-3-en-1-one with molecular oxygen in the presence of a solvent, of a base and of a catalyst of the formula I 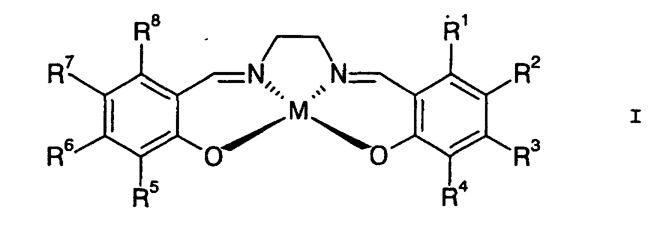 where
R¹, R², R³, R⁴, R⁵, R⁶, R⁷ and R⁸ are, independently of one another, hydrogen, halogen, NO₂, COR⁹, OCOR⁹, COOR⁹_{,} SO₂R⁹ or SO₃R⁹, where
R⁹ is hydrogen or a C₁-C₄-alkyl radical,
M is Mn(II), Mn(III)⁽⁺⁾X⁽⁻⁾, Co(II), Co(III)⁽⁺⁾X⁽⁻⁾, Fe(II), Fe(III)⁽⁺⁾X⁽⁻⁾, Cu(II) or Ru(II), where
X⁽⁻⁾ is a negatively charged counter ion for metals in oxidation state III,
which process is carried out in the presence of one or more acetate salts of the general formula II
(R¹⁰R¹¹R¹²C-COO⁽⁻⁾)ₘ Y^{(m+)} (II)
as additive, where
R¹⁰, R¹¹ and R¹² are, independently of one another, hydrogen, F, Cl, Br, I or a C₁-C₄-alkyl radical,
Y is NH₄⁺ or a singly to quadruply charged metal cation of the 1st to 4th main group and
m is 1, 2, 3 or 4.

2. A process as claimed in claim 1, wherein NH₄OAc, LiOAc, NaOAc, KOAc or a mixture of these acetates is used as additive.

3. A process as claimed in either of claims 1 or 2, wherein a solvent with complexing properties is used as solvent.

4. A process as claimed in either of claims 1 or 2, wherein dimethylformamide (DMF), N-methylpyrrolidone (NMP) or dimethylacetamide (DMA) is used as solvent.

5. A process as claimed in any of claims 1 to 4, wherein a trialkylamine with 3 to 20 C atoms is used as base.

6. A process as claimed in any of claims 1 to 4, wherein tripropylamine is used as base.

7. A process as claimed in claim 1 or 2, wherein dimethylformamide (DMF) or dimethylacetamide (DMA) is used as solvent in combination with tripropylamine as base.

8. A process as claimed in claim 1, which is carried out in the presence of a catalyst of the formula Ia 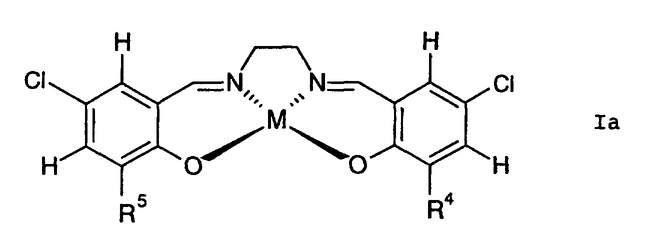 where
R⁴ and R⁵ are, independently of one another, hydrogen or Cl
M is Mn(II) or Mn(III)⁽⁺⁾ Cl⁽⁻⁾,
using dimethylformamide (DMF) or dimethylacetamide (DMA) as solvent and
tripropylamine as base and
with the addition of NH₄OAc, LiOAc, NaOAc or or a mixture of these acetates as additive.

9. A process as claimed in claim 1, which is carried out in an inverse reaction by feeding the precursor into the reaction mixture comprising the solvent, the base and the catalyst.

## Revendications

1. Procédé pour la préparation de 3,5,5-triméthylcyclohex-2-ène-1,4-dione par oxydation de 3,5,5-triméthylcyclohex-3-ène-1-one avec de l'oxygène moléculaire en présence d'un solvant, d'une base et d'un catalyseur de formule générale I, 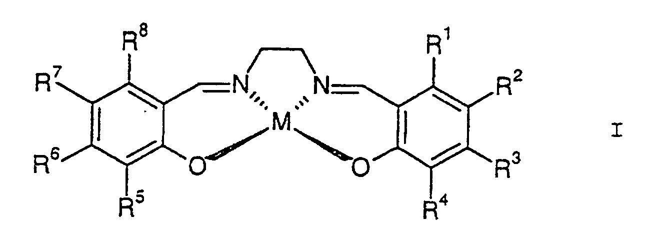 où
R¹, R², R³, R⁴, R⁵, R⁶, R⁷ et R⁸ désignent, indépendamment l'un de l'autre, hydrogène, halogène, NO₂, COR⁹, OCOR⁹, COOR⁹, SO₂R⁹ ou SO₃R⁹, tandis que
R⁹ désigne l'hydrogène ou un reste alkyle en C₁-C₄,
M représente Mn(II), Mn(III)⁽⁺⁾X⁽⁻⁾, Co(II), Co(III)⁽⁺⁾X⁽⁻⁾ , Fe(II), Fe(III)⁽⁺⁾X⁽⁻⁾ , Cu(II) ou Ru(II), où
X⁽⁻⁾ représente pour les métaux dans l'état d'oxydation (III) un contre-ion chargé négativement,
**caractérisé par le fait qu'**on conduit le procédé en présence d'un ou plusieurs sels acétates de formule générale II
(R¹⁰R¹¹R¹²C-COO⁽⁻⁾ₘ Y^{(m+)} (II)
à titre d'additif, tandis que
R¹⁰, R¹¹ et R¹² désignent, indépendamment l'un de l'autre, l'hydrogène, F, Cl, Br, I ou, un reste alkyle en C₁-C₄,
Y représente NH₄⁺ ou un cation de métal du 1^{er} au 4^{ème} groupe principal, chargé une à quatre fois, et
m vaut 1, 2, 3 ou 4.

2. Procédé selon la revendication 1, **caractérisé par le fait qu'**on utilise comme additif NH₄OAc, LiOAc, NaOAc, KOAc ou un mélange de ces acétates.

3. Procédé selon l'une des revendications 1 ou 2, **caractérisé par le fait qu'**on utilise comme solvant un solvant ayant des propriétés complexantes.

4. Procédé selon l'une des revendications 1 ou 2, **caractérisé par le fait qu'**on utilise comme solvant le diméthylformamide (DMF), la N-méthylpyrrolidone (NMP) ou le diméthylacétamide (DMA).

5. Procédé selon l'une des revendications 1 à 4, **caractérisé par le fait qu'**on utilise comme base une trialkylamine ayant 3 à 20 atomes de carbone.

6. Procédé selon l'une des revendications 1 à 4, **caractérisé par le fait qu'**on utilise comme base la tripropylamine.

7. Procédé selon la revendication 1 ou 2, **caractérisé par le fait qu'**on utilise comme solvant le diméthylformamide (DMF) ou le diméthylacétamide (DMA) en combinaison avec la tripropylamine comme base.

8. Procédé selon la revendication 1, **caractérisé par le fait qu'**on conduit le procédé en présence d'un catalyseur de formule générale Ia 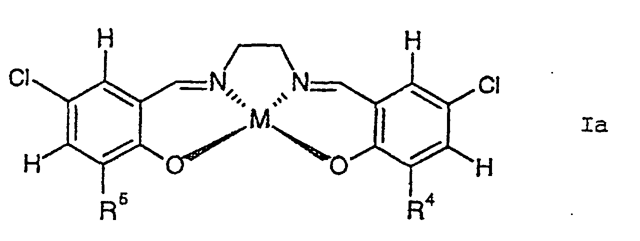 où
R⁴ et R⁵ représentent, indépendamment l'un de l'autre, l'hydrogène ou Cl,
M désigne Mn(II) ou Mn(III)⁽⁺⁾Cl⁽⁻⁾,
avec utilisation de diméthylformamide (DMF) ou de diméthylacétamide (DMA) comme solvant, de tripropylamine comme base, et
avec addition de NH₄OAc, LiOAc, NaOAc ou d'un mélange de ces acétates comme additif.

9. Procédé selon la revendication 1, **caractérisé par le fait qu'**on effectue le procédé par conduite inverse de la réaction, par introduction de l'éduit dans le mélange de réaction, contenant le solvant, la base et le catalyseur.
